# EUROPEAN PATENT SPECIFICATION

(11) **EP 0 717 849 B1**
(45) Date of publication and mention of the grant of the patent: **20.05.1998**
(21) Application number: 94926697.7
(22) Date of filing: 09.09.1994
(51) Int. Cl.: G01N 33/68, G01N 33/566, G01N 33/94

(54) **ASSAY SYSTEMS USING THE CNTF SIGNAL TRANSDUCTION PATHWAY**
TESTSYSTEM UNTER VERWENDUNG DES CNTF-SIGNALTRANSDUKTIONSWEGES
SYSTEMES DE DOSAGE DANS LESQUELS LA VOIE DE TRANSDUCTION DU SIGNAL DU FACTEUR NEUROTROPHIQUE CILIAIRE (CNTF) EST UTILISEE

(30) Priority: 09.09.1993 US 118968
(43) Date of publication of application: 26.06.1996
(73) Proprietor: REGENERON PHARMACEUTICALS, INC., Tarrytown, NY 10591-6707 (US); ST. JUDE CHILDREN'S RESEARCH HOSPITAL, Memphis, TN 38101-0318 (US)
(72) Inventor: STAHL, Neil, Carmel, NY 10512 (US); YANCOPOULOS, George, D., Yorktown Heights, NY 10598 (US); IHLE, James, N., Memphis, TN 38120 (US)
(74) Representative: Goldin, Douglas Michael
(86) International application number: US9410163
(87) International publication number: WO9507467

(56) References cited:
- WO-A-91/19009
- JOURNAL OF BIOLOGICAL CHEMISTRY, vol.269, no.15, 15 April 1994, WASHINGTON DC USA pages 11648 - 11655 T.G. BOULTON ET AL. 'CILIARY NEUROTROPHIC FACTOR/LEUKEMIA INHIBITORY FACTOR/INTERLEUKIN 6/ONCOSTATIN M FAMILY OF CYTOKINS INDUCES TYROSIN PHOSPHORYLATION OF A COMMON SET OF PROTEINS OVERLAPPING THOSE INDUCED BY OTHER CYTOKINES AND GROWTH FACTORS.'

## Description

### 1. INTRODUCTION

The present invention provides for assay systems that may be used to measure the ability of a test agent to act as an agonist or antagonist of ciliary neurotrophic factor (CNTF) as well as other cytokines, such as interleukin-6 (IL-6) oncostatin M (OSM) and leukemia inhibitory factor (LIF) that share receptor components with CNTF. The invention further provides for assay systems that may be used to detect agents that act as agonists or antagonists of CNTF and factors that share receptor components with CNTF.

### 2. BACKGROUND OF THE INVENTION

### 2.1. CILIARY NEUROTROPHIC FACTOR

Ciliary neurotrophic factor (CNTF), as its name implies, is a protein that is specifically required for the survival of embryonic chick ciliary ganglion neurons in vitro [Manthorpe et al., J. Neurochem. 34:69-75 (1980)]. CNTF has been cloned and synthesized in eukaryotic as well as bacterial expression systems, as described in International Application WO-A-91 04029.

Over the past decade, a number of biological effects have been ascribed to CNTF in addition to its ability to support the survival of ciliary ganglion neurons. CNTF is believed to induce the differentiation of bipotential glial progenitor cells in the perinatal rat optic nerve and brain [Hughes et al., Nature 335:70-73 (1988)]. Furthermore, it has been observed to promote the survival of embryonic chick dorsal root ganglion sensory neurons [Skaper and Varon, Brain Res. 389:39-46 (1986)].

Several novel activities of CNTF have also been discovered, including its ability to support the survival and differentiation of motor neurons and hippocampal neurons, and to increase the rate of hippocampal astrocyte proliferation (International Application No. PCT/US 90/05241, supra).

### 2.2. CILIARY NEUROTROPHIC FACTOR RECEPTOR

The CNTF receptor (CNTFR or CNTFRα) has been cloned and expressed in eukaryotic cells, as described in International Application WO-A-91 19009.

The sequence of CNTFR reveals that, unlike most receptors which contain an extracellular domain, a hydrophobic transmembrane domain, and a cytoplasmic domain, CNTFR does not appear to have a cytoplasmic domain. Additionally, the transmembrane hydrophobic domain is proteolytically processed, with the mature form of CNTFR becoming anchored to the cell surface by an unconventional linkage, referred to as a glycophosphatidyl inositol (GPI) linkage (Id.). GPI-linked proteins such as CNTFR may be released from the cell surface through cleavage of the GPI anchor by the enzyme phosphatidylinositol-specific phospholipase C. Of other known receptor sequences, CNTFR is related to a number of receptors, referred to herein as the CNTF/IL-6/LIF receptor family, including IL-6, LIF, G-CSF and oncostatin M (OSM) [Bazan, Neuron 7:197-208 (1991); Rose and Bruce, Proc. Natl. Acad. Sci. 88: 8641-8645, (1991)], but appears to be most closely related to the sequence of the receptor for IL-6. However, IL-6 has not been shown to be a GPI-linked protein [e.g., Taga, et al.,, Cell 58:573-581 (1989); Hibi, et al., , Cell 63:1149-1157 (1989)].

The cloning, sequencing and expression of the CNTF receptor (CNTFR) led to the discovery that CNTFR and CNTF may form a complex that interacts with a membrane bound, signal transducing component, thus suggesting therapeutic activity of a soluble CNTF/CNTFR receptor complex (see, for example, WO-A-93/11253).

One such signal transducing component involved in high affinity binding of CNTF and the subsequent functional response of the cell has been identified as gp130, a β component common to the IL-6, Oncostatin M, LIF family of receptors [Fukunaga et al., EMBO J. 10: 2855-2865 (1991); Gearing et al., EMBO J. 10: 2839-2848 (1991); Gearing et al. Science 255: 1434-1437 (1992); Ip, et al., Cell 69: 1121-1132 (1992)]. A further β component identified as being involved in binding and signal transduction in response to LIF (LIFRβ) appears to be the same or similar to a β component necessary for response to CNTF. (As a consequence of the identification of β components necessary for binding and signal transduction of CNTF, what was originally generally termed CNTFR is currently referred to as CNTFRα).

IL-6 is a pleiotropic cytokine which acts on a wide variety of cells, exerting growth promotion and inhibition and specific gene expression sometimes accompanied by cellular differentiation; its has been implicated as being involved in several diseases including inflammation, autoimmunities and lymphoid malignancies [Kishimoto, et al. Science 258:593-(1992). LIF, G-CSF and OSM are all broadly acting factors that, despite having unique growth-regulating activities, share several common actions with IL-6 during hemopoiesis as well as in other processes. For example, all can inhibit the proliferation and induce the differentiation of the murine myeloid leukemia cell line, M1 [[Rose, 1991 #1377]Rose and Bruce, Proc. Natl. Acad. Sci. 88: 8641-8645 (1991)]. LIF and Oncostatin M induced tyrosine phosphorylations and gene activations in neuronal cells which are indistinguishable from responses induced by CNTF (Ip, et al. 1992, Cell 69:1121-1132). In WO-A 93/11253 entitled "Cell Free Ciliary Neurotrophic Factor/Receptor Complex", CNTF and CNTFRα are combined to form a stable, biologically active complex that can be used as a differentiation or proliferation factor in cell types that express signal transducing receptor components belonging to the CNTF/IL-6/LIF receptor family. Signal transduction can be initiated by treatment of cells expressing both gp130 and LIFRβ with a soluble CNTF/CNTFRα complex. Alternatively, target cells not previously responsive to CNTF, but expressing LIFRβ and gp130 (such as LIF-responsive cells) can be made responsive to CNTF by attaching the CNTFRα to the cells and subsequently treating with CNTF.

Although the events surrounding CNTF binding and receptor activation have recently been elucidated [Davis, et al. Science 253:59-63 (1991); Ip, et al., Cell 69: 1121-1132 (1992); Stahl et al., Cell 74:587-590 (1993); Davis et al., Science 260:1805-1018 (1993)], the mechanism by which signal transduction is initiated inside the cell is more poorly understood. Like the other distantly related receptors for the extended cytokine family- which includes Interleukin (IL)-3, IL-5, GM-CSF, G-CSF, EPO, GH, and the interferons [(Bazan, J. F. Proc. Natl. Acad. Sci. USA 87:6934-6938 (1990); Bazan, J. F. Neuron 7:197-208 (1991)]- the CNTF receptor β subunits gp130 and LIFRβ do not have protein tyrosine kinase domains in their cytoplasmic regions (Hibi et al., Cell 63:1149-1157 (1990); Gearing et al., EMBO J. 10: 2839-2848 (1991). In spite of this, CNTF-induced dimerization of the β subunits somehow results in the rapid accumulation of a set of tyrosine phosphorylated proteins called the CLIPs [Ip, et al., Cell 69: 1121-1132 (1992)].

Although, as described above, two of the more prominent CLIPs were identified as the β subunits themselves, most of the others have yet to be characterized. The activation of cytoplasmic tyrosine kinase(s) appears to be essential for CNTF action since inhibitors that block the tyrosine phosphorylations also block subsequent downstream events such as gene inductions [Ip, et al., Cell 69: 1121-1132 (1992)].

A possible clue to the identity of the cytoplasmic tyrosine kinase(s) activated by the CNTF family of factors came from the finding that other distantly related cytokines resulted in the activation of the Jak/Tyk family of kinases (Firmbach-Kraft et al., Oncogene 5:1329-1336 (1990); Wilks et al., Mol. Cell. Biol 11:2057-2065(1991); Harpur et al., Oncogene 7:1347-1353 (1992). This family of nonreceptor cytoplasmic protein tyrosine kinases consists of 3 known members - Jak1, Jak2, and Tyk2 - which are all equally related to each other and share the unusual feature of having two potential kinase domains and no Src homology 2 (SH2) domains. Elegant studies involving complementation of a genetic defect in a cell line unresponsive to IFNa resulted in the identification of Tyk2 as a required component of the IFNα signaling cascade [(Velazquez et al., Cell 70:313-322 (1992)]. More recently, the receptors for cytokines such as EPO, GM-CSF, and GH were shown to associate with and activate Jak2 [Argetsinger et al., Cell 74:237-244 (1993); Witthuhn et al., Cell 74:227-236 (1993)]. The kinase was shown to bind to the membrane proximal cytoplasmic region of the receptor, and mutations of this region that prevented Jak2 binding also resulted in the loss of EPO induced proliferation, suggesting that Jak2 plays a critical role in EPO signaling. Jak1 has not been reported to be significantly activated by any of these receptor systems.

The identification of hemopoietic factors that share receptor components with CNTF would enable the utilization of CNTF and its specific receptor components for activation of targeted cells that are normally responsive to such hemopoietic factors.

### 3. SUMMARY OF THE INVENTION

The present invention provides for assay systems that may be used to detect and/or measure CNTF activity, or activity of other cytokines, such as LIF, Oncostatin M or IL-6 that utilize receptor components in common with CNTF (hereinafter the "CNTF receptor family"). The present invention also provides for assay systems that may be used to identify agents that act as agonists or antagonists of members of the CNTF receptor family.. It is based, in part, on the discovery that CNTF receptor family members utilize, as part of their signal transduction pathway, the CLIP signal transduction pathways and the Jak family of kinases.

The present invention provides a method of measuring the ability of a test agent to act as an agonist or antagonist of a member of the CNTF receptor family comprising;
(a) providing a cell that co-expresses the β-receptor component(s) of the CNTF receptor family member and a member of the Jaks;
(b) contacting the cell of step (a) with the test agent;
(c) determining the amount of tyrosine phosphorylation of the member of the Jaks in said cell; and
(d) comparing the amount of tyrosine phosphorylation determined in step (c) with the tyrosine phosphorylation of the same member of the Jaks in a cell of step (a) that has not been exposed to said test agent.

The invention further provides a method of detecting agonist or antagonist activity versus a member of the CNTF receptor family comprising:
(a) providing a cell that co-expresses the β-receptor component(s) of the CNTF receptor family member and a member of the Jaks;
(b) contacting the cell of step (a) with a test agent in the presence of the α-receptor components of the CNTF receptor family member;
(c) determining the amount of tyrosine phosphorylation of the member of the Jaks in said cell, and
(d) comparing the amount of tyrosine phosphorylation determined in step (c) with the tyrosine phosphorylation of the same member of the Jaks in a cell of step (a) that has been contacted with the CNTF receptor family member to determine whether the test agent acts as an agonist or antagonist of a member of the CNTF receptor family.

The invention yet further provides a cell genetically engineered to co-express a β-receptor component of the CNTF receptor family and a member of the Jaks which cell does not naturally co-express the β-receptor component and a Jaks.

### 4. DESCRIPTION OF THE FIGURES

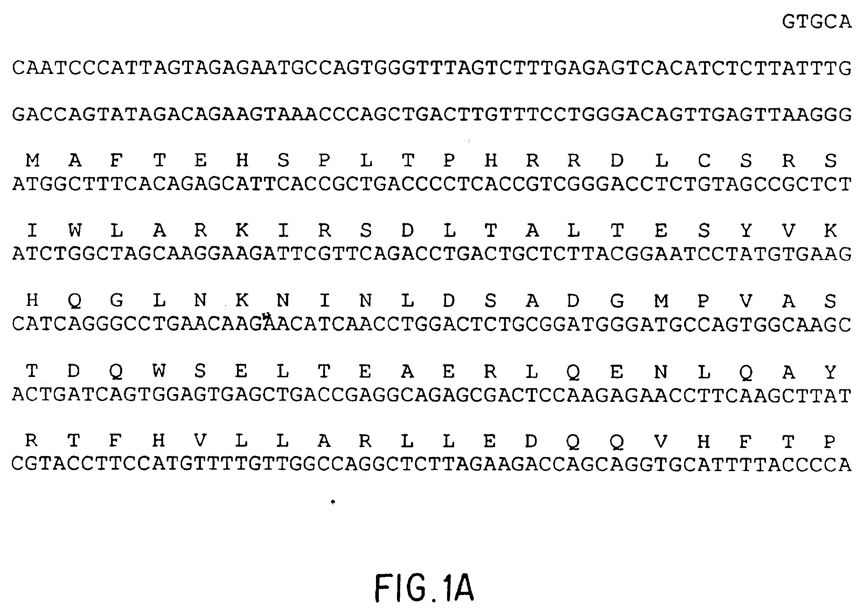

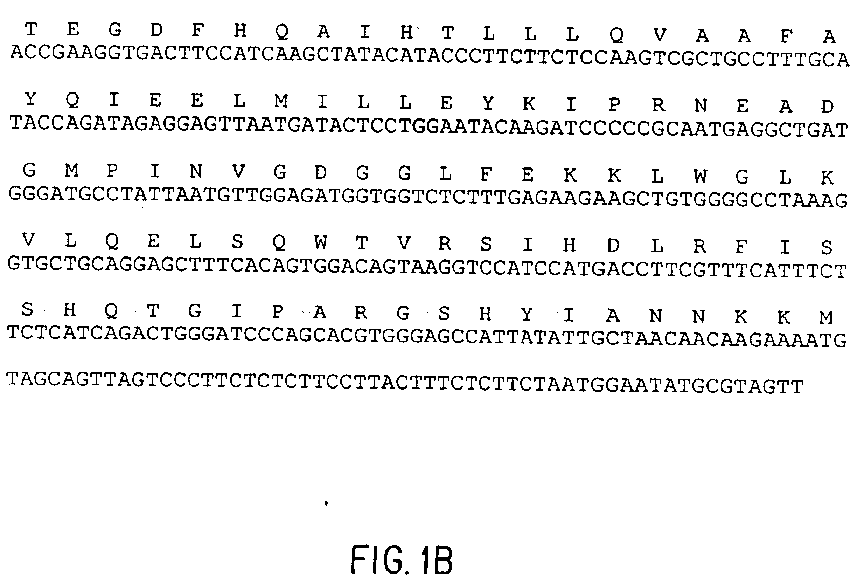

FIGURE 1. Nucleic acid sequence (SEQ ID NO:1) of human CNTF and the deduced amino acid sequence (SEQ ID NO:2).

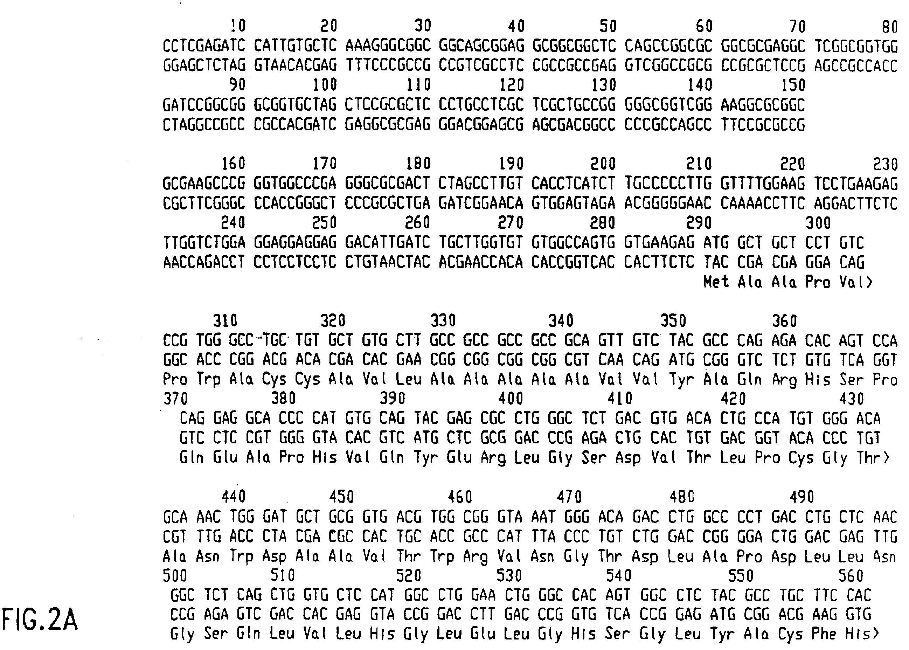

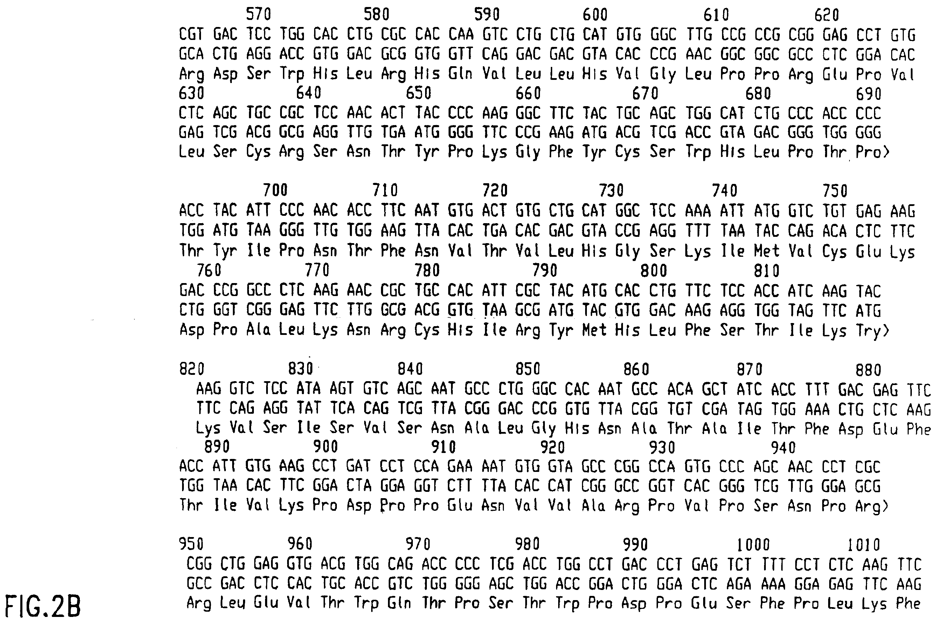

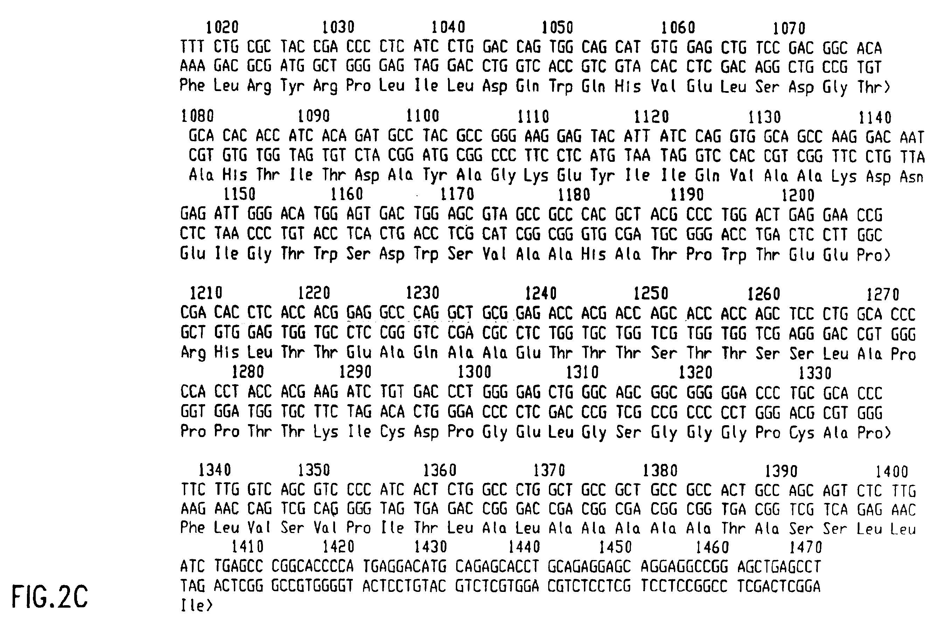

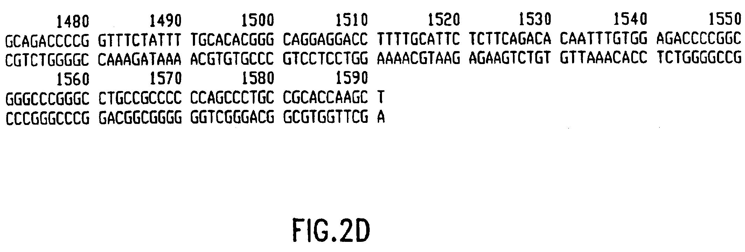

FIGURE 2. Nucleic acid sequence (SED ID NO:3) of CNTFR encoding cDNA and deduced amino acid sequence (SEQ ID NO:4).

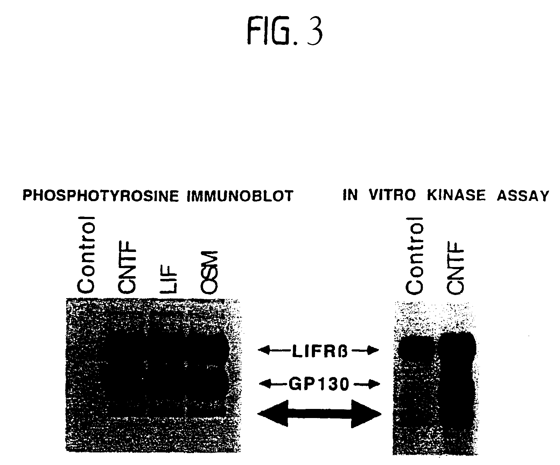

Figure 3: A 130 kDa protein and tyrosine kinase activity copurify with CNTF receptor complexes. EW-1 cells were stimulated with the indicated factor, then the cells were lysed in Brij 96 detergent and immunoprecipitated with a-LIFRb. Samples in the left panel were immunoblotted with a-phosphotyrosine, while those in the right were tested for in vitro kinase activity as described in the Experimental section.

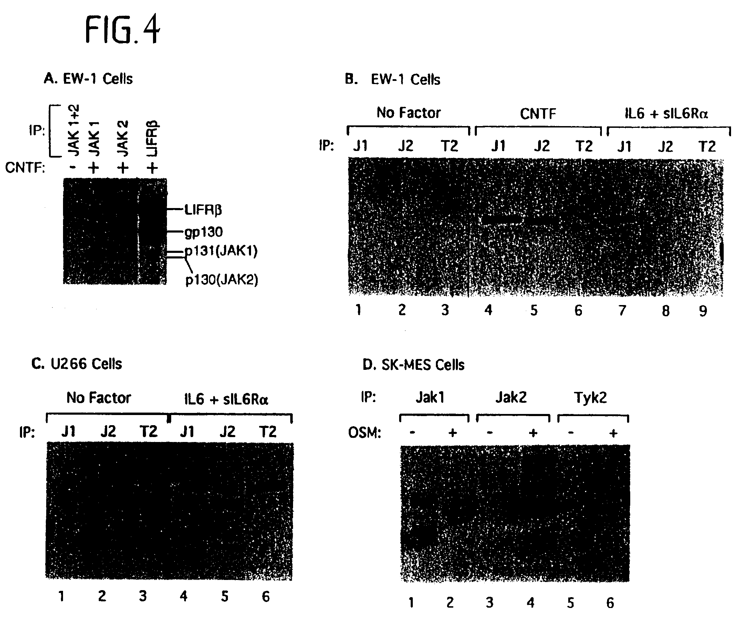

Figure 4: Jak1, Jak2, and Tyk2 become tyrosine phosphorylated in response to the CNTF family of factors. Either EW-1 (Panels A & B), U266 (Panel C), or SK-MES cells (Panel D) were stimulated with the indicated factor, immunoprecipitated with antisera against LIFRβ, Jak1 (J1), Jak2 (J2), or Tyk2 (T2), then immunoblotted with anti-phosphotyrosine.

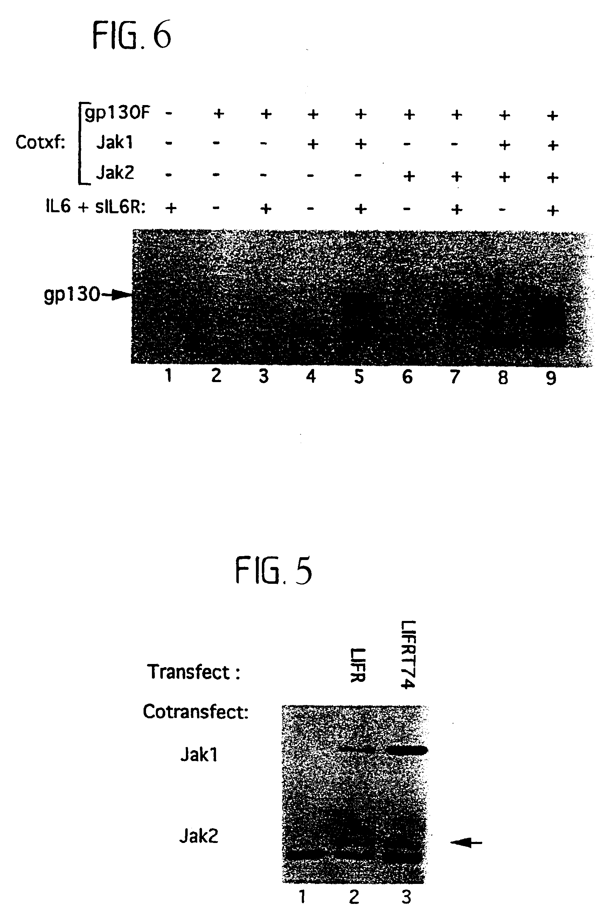

Figure 5: LIFRβ binds Jak1 and Jak2 in the absence of factors. COS cells were cotransfected with plasmids encoding Jak1 or Jak2, along with those encoding either epitope-tagged LIFRβ-myc3 (LIFR) or a truncated version of LIFRβ encoding only 74 amino acids of the cytoplasmic domain followed by the triple-myc tag. (LIFRT74). Following immunoprecipitation with the a-myc monoclonal 9E10, the samples were immunoblotted with antisera against Jak1 (top panel) or Jak2 (lower panel). The arrow in the bottom panel indicates the Jak2 band which migrates more slowly than the prominent nonspecific background band.

Figure 6: Coexpression of either Jak1 or Jak2 with gp130 in COS enhance IL-6 dependent tyrosine phosphorylation of gp130. COS cells were cotransfected with 0.5 mg of Jak1 or Jak2 encoding plasmid as well as 10 mg of gp130FLAG encoding plasmid, then stimulated 48 hours later with IL6 + sIL6Rα as indicated. Cell lysates were immunoprecipitated with a-FLAG monoclonal antibodies (IBI), and immunoblotted with anti-phosphotyrosine.

### 5. DETAILED DESCRIPTION ASSAY SYSTEMS

The present invention provides for assay systems and methods that may be used to detect and/or measure CNTF activity or to identify agents that act as agonists or antagonists of CNTF or other cytokine members of the CNTF receptor family. This activity may be construed to be the biological activity of CNTF or the related cytokine, or of other, heretofore unidentified factors which utilize receptor components or signal transduction pathways in common with the CNTF receptor family. The biological activities of CNTF have been described in International Application WO-A-91 04029 ; of IL-6 in Kishimoto, et al. Science 258:593 (1992); of OSM in Rose and Bruce, Proc. Natl. Acad. Sci. USA, 88:8641-8645 (1991); Grove, et al. J. Biol. Chem. 266:18194-18199 (1991); Liu, et al., J. Biol. Chem. 267:16763-16766 (1992) and of LIF in Chang and Gough, Focus 14:6-9 (1991); Escary, et al. Nature 363:361-363 (1993). Henceforth, both CNTF receptor family members and agents which act as agonists or antagonists of such activity will be referred to as test agents.

Accordingly, the present invention provides for methods of detecting or measuring the activity of a test agent. Each such method involves, at a minimum, a cell that expresses the β-component(s) of the receptor as well as a signal transduction component selected from the group consisting of Jak1, Jak2 and Tyk2 or any other signal transduction Jaks component, that is determined to be phosphorylated in response to a member of the CNTF receptor family.

A cell that expressed the β-receptor component(s) and signal transduction component(s) described herein may either do so naturally or be genetically engineered to do so. For example, Jak1-encoding nucleic acid sequences obtained as described in section 6.1, infra, may be introduced into a cell by transduction, transfection, microinjection, electroporation, via a transgenic animal, etc., using any known method known in the art. See for example, the transfection of COS cells as described in section 6.1.2, infra.

According to the invention, the cells are exposed to a test agent and the tyrosine phosphorylation of β-the signal transduction component(s) are compared to the tyrosine phosphorylation of the same component(s) in the absence of the test agent.

In another embodiment of the invention, the tyrosine phosphorylation that results from contacting the above cells with the test agent is compared to the tyrosine phosphorylation of the same cells exposed to the CNTF receptor family member. In such assays, the cell must either express the extracellular receptor (α-component) or the cells may be exposed to the test agent in the presence of the soluble receptor component. Thus, for example, in an assay system designed to identify agonists or antagonists of CNTF, the cell may express the α-component CNTFRα, the β-components gp130 and LIFRβ and a signal transducing component such as Jak1. The cell is exposed to test agents, and the tyrosine phosphorylation of either the β-components or the signal transducing component is compared to the phosphorylation pattem produced in the presence of CNTF. Alternatively, the tyrosine phosphorylation which results from exposure to a test agent is compared to the phosphorylation which occurs in the absence of the test agent. Alternatively, an assay system, for example, for IL-6 may involve exposing a cell that expresses the β-component gp130 and a signal transducing protein such as Jak1, Jak2 or Tyk2 to a test agent in conjunction with the soluble IL-6 receptor (see section 6.2.4, infra.).

In another embodiment of the invention, no α-receptor components are present. Such assay systems are useful to identify small molecules that are capable of initiating or blocking signal transduction absent binding to a cell surface receptor component.

### 6. EXAMPLE: THE JAK FAMILY OF KINASES ARE INVOLVED IN SIGNAL TRANSDUCTION BY THE CNTF FAMILY OF FACTORS

### 6.1 MATERIALS AND METHODS

### 6.1.1. REAGENTS

Antisera specific for LIFRβ (Stahl, et al., J. Biol Chem. 268:7628-7631 (1993) and gp130 (Davis et al., Science 260:1805-1808 (1993) have been described. The rabbit antiserum against Tyk2 was raised and purified against a portion of Tyk2 expressed as a glutathione-S-transferase (GST) fusion protein (Velazquez et al., Cell 70:313-322 (1992)). Expression plasmids appropriate for COS expression of epitope-tagged LIFRβ and gp130 were previously described (Davis et al., Science 260:1805-1808 (1993), except that the LIFRβ coding sequence was modified to contain 3 successive copies of the myc epitope to improve detectability. Full length cDNA for murine Jak1 and Jak2 were provided in the plasmid pRK5 (J. Ihle et al., unpublished).

### 6.1.2 METHODS

Cell lines were passaged and maintained as previously described (Ip et al., Cell 69:1121-1132 (1992). COS cell transfections were carried out by a DEAE protocol [Davis et al., Science 260:1805-1808 (1993)]. Plates of cells were starved in serum-free RPMI medium for 2-4 hours, then stimulated with 50 ng/mL of the indicated factor for 5 minutes. Cells were harvested and lysed as previously described [Stahl, et al., J. Biol Chem. 268:7628-7631 (1993)], except that 1% Brij 96 (Sigma) or 1% NP-40 (Boehringer) was used as indicated. Immunoprecipitation, electrophoresis, and anti-phosphotyrosine immunoblotting with monoclonal antibody 4G10 (Upstate Biotechnology) and detection via enhanced chemiluminescence (Amersham) was carried out as previously described (Id). For in vitro kinase assays, the washed beads were incubated for 15 min at room temperature in 20 mM Hepes (pH 7.2), 10 mM MnCl2, 30 mM sodium orthovanadate and 10 mCi of [g-³²P]ATP (NEN Dupont). Electrophoresis sample buffer was added and the samples were boiled, subjected to SDS PAGE, and electroblotted to PVDF. The membrane was then incubated in 1 M NaOH at 65°C for 60 min to destroy serine and threonine phosphate before autoradiography.

### 6.2. RESULTS

### 6.2.1. CNTF-INDUCED RESPONSES ARE ASSOCIATED WITH A 130kDa PROTEIN

Following addition of CNTF, a receptor complex forms that consists of CNTF, CNTFRα, gp130, and LIFRβ. Immunoprecipitation (IP) of the receptor complex with antibodies against LIFRβ (Figure 3) or gp130 (not shown) following cell lysis in the detergent Brij 96 results in the copurification of a 130 kDa protein that is tyrosine phosphorylated. LIF and OSM, which also bind to and heterodimerize gp130 and LIFRβ [Gearing et al., Science 255:1434-1437 (1992); Baumann et al., J. Biol. Chem. 268:8414-8417 (1993); Davis et al., Science 260:1805-1808 (1993)], also show association and tyrosine phosphorylation of a protein with an identical appearance (Figure 3). The purified receptor complex also shows associated protein tyrosine kinase activity in vitro giving rise to tyrosine phosphorylation of both gp130 and LIFRβ, as well as the associated 130 kDa protein. Tyrosine kinase activity is also associated with LIFRβ in the absence of CNTF, although the 130 kDa protein is either not present or not significantly phosphorylated in the absence of the factor. Other experiments showing that this in vitro kinase activity has the same sensitivity to staurosporine as that observed upon addition of CNTF to intact cells suggested that this associated tyrosine kinase activity is relevant to that which is required in the cell to mediate CNTF-induced responses. Furthermore, the 130 kDa protein appears to be a good candidate for this kinase since lysis of the cells in NP-40 does not give copurification of either the 130 kDa protein or tyrosine kinase activity (not shown).

### 6.2.2. CNTF AND RELATED FACTORS INDUCE TYROSINE PHOSPHORYLATION OF JAK 1, JAK2 and TYK2

Experiments using specific antisera raised against portions of Jak1, Jak2, or Tyk2 reveal that all 3 of these kinases can become tyrosine phosphorylated following stimulation by CNTF, LIF, OSM, and IL6. Figure 4A shows that CNTF induces tyrosine phosphorylation of both Jak1 and Jak2 in EW1 cells, and these proteins appear to comigrate with 130 and 131 kDa proteins that copurify with the receptor complex immunoprecipitated with a-LIFRβ. Furthermore, the addition of IL6 + sIL6Rα (Figure 4B), as well as LIF and OSM (not shown) to EW-1 cells also results in phosphorylation of Jak1 and Jak2 but not Tyk2. In contrast, IL6 stimulated U266 cells give tyrosine phosphorylation of Tyk2 and Jak1 without apparent change in the phosphorylation status of Jak2. OSM treated SK-MES cells reveal tyrosine phosphorylation of primarily Jak2, with smaller changes in Tyk2 and Jak1. In each of these cases, tyrosine phosphorylation of the Jaks or Tyk2 is associated with an increase in their in vitro tyrosine kinase activity (not shown). These results stand in contrast to previous results showing that stimulation with GM-CSF, EPO, G-CSF, IFN-γ, or IL-3 only result in tyrosine phosphorylation of Jak2 [(Argetsinger et al., Cell 74:237-244 (1993); Witthuhn et al., Cell 74:227-236 (1993)]. We conclude from these experiments that the CNTF family of factors can activate Jak1, Jak2, and Tyk2, although there is some variability in which Jak/Tyk family member is activated in a particular cell.

### 6.2.3. THE JAKS ASSOCIATE WITH CNTF β RECEPTOR COMPONENTS

Transient transfections in COS cells were used to determine whether the Jaks could associate with the β receptor components In the absence of factors. These experiments used carboxy terminally epitope-tagged versions of LIFRβ containing the 10 amino acid portion of c-myc that is recognized by the monoclonal antibody 9E10 [Davis et al., Science 253:59-63 (1991)]. COS cells were cotransfected with appropriate expression vectors encoding full length versions of LIFRβ and Jak1 or Jak2, and Brij 96 lysates were immunoprecipitated with 9E10 and then blotted with the antisera against either Jak1 or Jak2 (Figure 5). These experiments show that either Jak can associate with LIFRβ in the absence of any added ligand. Furthermore, a truncated version of LIFRβ which retains only the first 76 amino acids of the cytoplasmic domain is fully capable of binding to Jak1 and Jak2 as well. This implicates the membrane proximal region of LIFRβ as the Jak binding domain, which is consistent with the homology between this region of the receptor with those in gp130 and EPOR that have been shown to be required for signal transduction upon factor binding [Murakami et al., Science 260:11349-11353 (1991); Witthuhn et al., Cell 74:227-236 (1993)].

### 6.2.4 COTRANSFECTION WITH RECEPTOR β-COMPONENTS AND JAKS RESULTS IN LIGAND INDUCED FUNCTIONAL RESPONSE

Further experiments in COS cells were undertaken to establish whether cotransfection of the receptor β-components with the Jaks could reconstruct a ligand-induced functional response. Epitope-tagged gp130FLAG and IL6 were chosen for these experiments, since gp130 homodimerizes and becomes tyrosine phosphorylated in response to IL6 + soluble IL6Rα, obviating the need for cotransfection with LIFRβ [Murakami et al., Proc. Natl. Acad. Sci. 88:11349-11353 (1993); Davis et al., Science 260:1805-1808 (1993)]. Following stimulation with IL6 + slL6Rα, neither mock transfected (lane 1) nor gp130FLAG transfected COS cells (lanes 2-3) revealed substantial tyrosine phosphorylation of gp130 following immunoprecipitation with anti-FLAG and α-PTyr immunoblotting (Figure 6). In contrast, cotransfection with either Jak1 (lanes 4-5), Jak2 (lanes 6-7), or both Jak1 and Jak2 (lanes 8-9) gives rise to a substantial increase in the induced tyrosine phosphorylation of gp130 upon stimulation with IL6 + sIL6Rα.

### 6.3 DISCUSSION

Altogether, these results indicate that the Jaks can associate with the CNTF receptor β components, and become tyrosine phosphorylated in response to CNTF, LIF, IL6, or OSM, with concomitant activation of the tyrosine kinase. This most likely occurs through transphosphorylation as ligand-induced hetero- or homo-dimerization of the β components brings their bound Jaks into close apposition [Stahl and Yancopoulos, Cell 74:587-590 (1993)]. The functional reconstruction in COS cells of ligand-induced tyrosine phosphorylation of gp130 upon cotransfection with either Jak1 or Jak2 is consistent with the notion that Jak1, Jak2, or Tyk2 can function as the first kinases activated inside the cell upon receptor β subunit dimerization, thus placing the Jak family of kinases as the most proximal intracellular step in mediating signal transduction of the CNTF family of factors.

## Claims

1. A method of measuring the ability of a test agent to act as an agonist or antagonist of a member of the ciliary neurotrophic factor (CNTF) receptor family comprising;
(a) providing a cell that co-expresses the β-receptor component(s) of the CNTF receptor family member and a member of the Jaks;
(b) contacting the cell of step (a) with the test agent;
(c) determining the amount of tyrosine phosphorylation of the member of the Jaks in said cell; and
(d) comparing the amount of tyrosine phosphorylation determined in step (c) with the tyrosine phosphorylation of the same member of the Jaks in a cell of step (a) that has not been exposed to said test agent.

2. A method according to claim 1 wherein said CNTF receptor family member is selected from CNTF, LIF, OSM and IL-6.

3. A method according to claim 1 or 2 wherein said β-component is gp130.

4. A method according to claim 1 or 2 wherein said β-component is LIFRβ.

5. A method according to claim 1 or 2 wherein the cell of step (a) co-expresses both gp130 and LIFRβ as β-receptor components.

6. A method according to any one of the preceding claims wherein said member of the Jaks is selected from Jak1, Jak2 and Tyk2.

7. A method according to any one of the preceding claims, wherein said cell of step (a) further expresses the α-receptor component of the CNTF receptor family member.

8. A method according to any one of the preceding claims wherein the α-receptor components of the CNTF receptor family member are added in soluble form in conjunction with said test agent.

9. A method according to claim 7 or 8 wherein said α-component is CNTRFα or IL-6Rα.

10. A method of detecting agonist or antagonist activity versus a member of the ciliary neurotrophic factor (CNTF) receptor family comprising:
(a) providing a cell that co-expresses the β-receptor component(s) of the CNTF receptor family member and a member of the Jaks;
(b) contacting the cell of step (a) with a test agent in the presence of the α-receptor components of the CNTF receptor family member;
(c) determining the amount of tyrosine phosphorylation of the member of the Jaks in said cell, and
(d) comparing the amount of tyrosine phosphorylation determined in step (c) with the tyrosine phosphorylation of the same member of the Jaks in a cell of step (a) that has been contacted with the CNTF receptor family member to determine whether the test agent acts as an agonist or antagonist of a member of the CNTF receptor family.

11. A cell genetically engineered to co-express a β-receptor component of the ciliary neurotrophic factor (CNTF) receptor family and a member of the Jaks which cell does not naturally co-express the β-receptor component and a Jaks.

12. A cell according to claim 11 which further expresses an α-receptor component of the CNTF receptor family.

13. A cell according to claim 11 or 12 which is cotransfected with the β-receptor component and a Jaks.

14. A cell according to any one of claims 11 to 13 which is a COS cell.

## Patentansprüche

1. Verfahren zur Messung der Fähigkeit eines Testmittels zur Wirkung als ein Agonist oder Antagonist eines Mitglieds der Rezeptorfamilie des cililären neurotrophen Faktors (CNTF), umfassend
(a) Bereitstellung einer Zelle, die den (die) β-Rezeptorbestandteil(e) des Mitglieds der CNTF-Rezeptorfamilie und ein Mitglied der Jak-Familie gemeinsam exprimiert;
(b) Inkontaktbringen der Zelle von Schritt (a) mit dem Testmittel;
(c) Bestimmung des Ausmaßes der Tyrosinphosphorylierung des Mitglieds der Jak-Familie in der Zelle und
(d) Vergleich des in Schritt (c) bestimmten Ausmaßes der Tyrosinphosphorylierung mit der Tyrosinphosphorylierung des gleichen Mitglieds der Jak-Familie in einer Zelle von Schritt (a), die dem Testmittel nicht ausgesetzt war.

2. Verfahren nach Anspruch 1, wobei das Mitglied der CNTF-Rezeptorfamilie ausgewählt ist aus CNTF, LIF, OSM und IL-6.

3. Verfahren nach Anspruch 1 oder 2, wobei die β-Komponente gp130 ist.

4. Verfahren nach Anspruch 1 oder 2, wobei die β-Komponente LIFRβ ist.

5. Verfahren nach Anspruch 1 oder 2, wobei die Zelle von Schritt (a) sowohl gp130 als auch LIFRβ als β-Rezeptorbestandteile gemeinsam exprimiert.

6. Verfahren nach einem der vorhergehenden Ansprüche, wobei das Mitglied der Jak-Familie ausgewählt ist aus Jak1, Jak2 und Tyk2.

7. Verfahren nach einem der vorhergehenden Ansprüche, wobei die Zelle von Schritt (a) außerdem den α-Rezeptorbestandteil des Mitglieds der CNTF-Rezeptorfamilie exprimiert.

8. Verfahren nach einem der vorhergehenden Ansprüche, wobei die α-Rezeptorbestandteile des Mitglieds der CNTF-Rezeptorfamilie in löslicher Form zusammen mit dem Testmittel zugegeben werden.

9. Verfahren nach Anspruch 7 oder 8, wobei der α-Bestandteil CNTRFα oder IL-6Rα ist.

10. Verfahren zum Nachweis einer Agonisten- oder Antagonisten-Aktivität gegenüber einem Mitglied der Rezeptorfamilie des ciliären neurotrophen Faktors (CNTF), umfassend,
(a) Bereitstellung einer Zelle, die den (die) β-Rezeptorbestandteil(e) des Mitglieds der CNTF-Rezeptorfamilie und ein Mitglied der Jak-Familie gemeinsam exprimiert;
(b) Inkontaktbringen der Zelle von Schritt (a) mit einem Testmittel in Gegenwart der α-Rezeptorbestandteile des Mitglieds der CNTF-Rezeptorfamilie;
(c) Bestimmung des Ausmaßes der Tyrosinphosphorylierung des Mitglieds der Jak-Familie in der Zelle und
(d) Vergleich des in Schritt (c) bestimmten Ausmaßes der Tyrosinphosphorylierung mit der Tyrosinphosphorylierung des gleichen Mitglieds der Jak-Familie in einer Zelle von Schritt (a), die mit dem Mitglied der CNTF-Rezeptorfamilie in Kontakt gebracht worden war, zur Bestimmung, ob das Testmittel als Agonist oder Antagonist eines Mitglieds der CNTF-Rezeptorfamilie wirkt.

11. Gentechnisch hergestellte Zelle zur gemeinsamen Expression eines β-Rezeptorbestandteils der Rezeptorfamilie ciliären neurotrophen Faktors (CNTF) und eines Mitglieds der Jak-Familie, wobei die Zelle natürlicherweise den β-Rezeptorbestandteil und ein Mitglied der Jak-Familie nicht gemeinsam exprimiert.

12. Zelle nach Anspruch 11, die außerdem einen α-Rezeptorbestandteil der CNTF-Rezeptorfamilie exprimiert.

13. Zelle nach Anspruch 11 oder 12, die mit dem β-Rezeptorbestandteil und einem Mitglied der Jak-Familie gemeinsam transfiziert ist.

14. Zelle nach einem der Ansprüche 11 bis 13, die eine COS-Zelle ist.

## Revendications

1. Procédé de mesure de l'aptitude d'un agent d'essai à agir en tant qu'agoniste ou antagoniste d'un membre de la famille des récepteurs du facteur neurotrophique ciliaire (CNTF), comprenant:
(a) la fourniture d'une cellule qui exprime simultanément le(s) composant(s) β de récepteur du membre de la famille des récepteurs CNTF et d'un membre des Jak;
(b) la mise en contact de la cellule de l'étape (a) avec l'agent d'essai;
(c) la détermination de la quantité de phosphorylation de résidus tyrosine du membre des Jak dans ladite cellule; et
(d) la comparaison de la quantité de phosphorylation de résidus tyrosine déterminée dans l'étape (c) avec la quantité de phosphorylation de résidus tyrosine du même membre des Jak dans une cellule de l'étape (a) qui n'a pas été exposée audit agent d'essai.

2. Procédé selon la revendication 1, dans lequel ledit membre de la famille des récepteurs CNTF est choisi parmi CNTF, LIF, OSM et IL-6.

3. Procédé selon la revendication 1 ou 2, dans lequel ledit composant β est gp130.

4. Procédé selon la revendication 1 ou 2, dans lequel ledit composant β est LIFRβ.

5. Procédé selon la revendication 1 ou 2, dans lequel la cellule de l'étape (a) exprime simultanément à la fois gp130 et LIFRβ en tant que composants du récepteur β.

6. Procédé selon l'une quelconque des revendications précédentes, dans lequel ledit membre des Jak est choisi parmi Jak1, Jak2 et Tyk2.

7. Procédé selon l'une quelconque des revendications précédentes, dans lequel ladite cellule de l'étape (a) exprime en outre le composant du récepteur α du membre de la famille des récepteurs CNTF.

8. Procédé selon l'une quelconque des revendications précédentes, dans lequel les composants de récepteur α du membre de la famille des récepteurs CNTF sont ajoutés sous forme soluble conjointement avec ledit agent d'essai.

9. Procédé selon la revendication 7 ou 8, dans lequel ledit composant α est CNTRFα ou IL-6Rα.

10. Procédé de détection d'activité agoniste ou antagoniste à l'égard d'un membre de la famille des récepteurs du facteur neurotrophique ciliaire (CNTF), comprenant:
(a) la fourniture d'une cellule exprimant simultanément le(s) composant(s) β de récepteur du membre de la famille des récepteurs CNTF et d'un membre des Jak;
(b) la mise en contact de la cellule de l'étape (a) avec un agent d'essai en présence des composants de récepteur α du membre de la famille des récepteurs CNTF;
(c) la détermination de la quantité de phosphorylation de résidus tyrosine du membre des Jak dans ladite cellule; et
(d) la comparaison de la quantité de phosphorylation de résidus tyrosine déterminée dans l'étape (c) avec la quantité de phosphorylation de résidus tyrosine du même membre des Jak dans une cellule de l'étape (a) qui a été mise en contact avec le membre de la famille des récepteurs CNTF, pour déterminer si les agents d'essai agissent en tant qu'agonistes ou antagonistes d'un membre de la famille des récepteurs CNTF.

11. Cellule transformée par génie génétique pour exprimer simultanément un composant de récepteur β de la famille des récepteurs du facteur neurotrophique ciliaire (CNTF) et un membre des Jak, laquelle cellule naturellement n'exprime pas simultanément le composant de récepteur β et une Jak.

12. Cellule selon la revendication 11, qui exprime en outre un composant de récepteur α de la famille des récepteurs CNTF.

13. Cellule selon la revendication 11 ou 12, qui est co-transfectée avec le composant de récepteur β et une Jak.

14. Cellule selon l'une quelconque des revendications à 13, qui est une cellule COS.
